Europäisches Patentamt

**European Patent Office**

Office européen des brevets

(11) Publication number: **0 070 632**
A2

# EUROPEAN PATENT APPLICATION

(21) Application number: 82303340.2

(22) Date of filing: 25.06.82

(51) Int. Cl.³: **C 12 N 15/00,** C 12 P 21/02, C 12 N 1/20 // C12R1/19

(30) Priority: 29.06.81 US 278331

(43) Date of publication of application: 26.01.83 Bulletin 83/4

(84) Designated Contracting States: **AT BE CH DE FR GB IT LI LU NL SE**

(71) Applicant: **CETUS CORPORATION, 600 Bancroft Way, Berkeley, California (US)**

(72) Inventor: **Bahl, Chander Prakash, 908 Norvell Street, El Cerrito California 94530 (US)**

(74) Representative: **Bizley, Richard Edward et al, BOULT, WADE & TENNANT 27 Furnival street, London EC4A 1PQ (GB)**

(54) Recombinant plasmids encoded for human preproinsulin and their use in producing human insulin.

(57) Recombinant plasmids are described that carry the structural gene for human proinsulin in expressible form niques using a single host organism. The human proinsulin by the application of genetic engineering techniques using a single host organism. The uman proinsulin structural gene is fused to a functional transport sequence. Host organisms transformed by the recombinant plasmids express a preproinsulin product that may, depending upon the nature of the host, be correctly processed to human proinsulin in vivo and transported across the host organism's cell wall. Alternatively, expressed human preproinsulin may be recovered from host cells and converted into human proinsulin. Following its preparation in vitro methods may be used to convert the human proinsulin to human insulin.

0070632

CLONING VECTOR FOR HUMAN PREPROINSULIN PRODUCTION,
ORGANISM AND CULTURE TRANSFORMED BY SAID VECTOR, HUMAN
PREPROINSULIN OR PROINSULIN PRODUCED BY SAID ORGANISM
OR CULTURE, HUMAN PROINSULIN PRODUCED FROM SAID
PREPROINSULIN, HUMAN INSULIN PRODUCED FROM SAID PROINSULIN,
AND A PROCESS FOR PREPARING SAID VECTOR, ORGANISM OR
CULTURE, PREPROINSULIN, PROINSULIN OR INSULIN.

This invention relates to molecular biology and more particularly to the so-called art of recombinant DNA. Specifically the invention relates to recombinant plasmids for producing human insulin.

The invention describes novel genetically engineered plasmids that carry a gene coding for human proinsulin. Representative plasmid, pJW2172, illustrates the invention and has been deposited with the American Type Culture Collection, Rockville, Maryland 20852; it has been awarded ATCC number 31891.

As is well known, insulin is a peptide hormone that exquisitely regulates a variety of vital metabolic events. In most animals insulin is synthesized as a single large precursor polypeptide, preproinsulin. A transport sequence and an internal peptide sequence are excised from preproinsulin during biosynthesis, yielding a biologically active molecule consisting of two peptide chains linked together by disulfide bonds. Excision of only the transport sequence yields a peptide molecule known as proinsulin. Proinsulins have now been isolated and characterized from a number of animals. See Steiner, D., "Peptide Hormone Precursors" in Peptide Hormones, Parsons, J.A.Editor, McMillan Press Limited, London (J.976) at pages 49-64.

The beta-cells of the islets of Langerhans produce insulin in vivo. Initially insulin is expressed as a fused polypeptide comprised of the proinsulin sequence plus a pre-sequence known as the transport or signal sequence. This transport sequence facilitates passage of the insulin molecule through the cellular membranes. In the higher eukaryotes, the preproinsulin is synthesized on ribosomes that are associated with the rough endoplasmic reticulum. See Permutt, M. and Kipnis, D.M., Proc. Nat.Acad.Sci., USA, 69:506-509 (1972). When it produced in vivo, the newly translated peptide is first transported intracellularly to the Golgi apparatus where it becomes incorporated within newly forming secretion granules. Exactly how the cell converts proinsulin to insulin is not known, but, conversion is believed to be initiated in either the Golgi apparatus or in the newly formed secretion granules. The secretion granules contain zinc and membrane bound proteases which are believed to participate in the conversion mechanism. The types of proteases can vary from species to species, perhaps to accommodate species specific amino acid substitutions in the insulin peptide. See Steiner, supra at 54.

The in vivo production of insulin ultimately results in the secretion of mature insulin molecules from which both the transport sequence and the C chains have been removed. Although it is known that insulin is liberated from the proinsulin in the secretion granules, and that the insulin transport sequence is involved in transport of the peptide across the cell membranes, neither the transport mechanism nor the processing mechanism are well understood.

Because some people fail to produce insulin at all, or fail to produce it in amounts sufficient to meet their own individual needs, it has long been necessary to find supplemental sources of insulin to administer to these people. Most of the supplemental insulin used to treat insulin deficient individuals has come from bovine and porcine pancreata obtained from animals killed at the slaughterhouses. Unfortunately, this animal insulin is immunogenic in some patients. Therefore it would be preferable to have human insulin to administer to those human patients who need it. However, until very recently there was no practical way to obtain enough human insulin to supply the needs of those patients who require it. Although it is possible to chemically synthesize human

- 3 -                                    0070632

insulin, on a commercial scale the cost of such production is prohibitive.

Fortunately, recent advances in recombinant DNA technology now make it possible to genetically engineer bacteria so that they can produce eukaryotic proteins such as insulin. For example, Goeddell, D. V., et al, Proc. Nat. Acad. Sci., USA 76:106-110 (1979) chemically synthesized the DNA sequences coding for the human A and B insulin chains. Recombinant techniques were then used to separately ligate each sequence to the 3' end of an E. coli beta-galactosidase gene. As expected, two hybrid proteins were obtained, each consisting of either the human A or B chain fused to the bacterial beta-galactosidase. The A and B chains were released from the fusion peptides by chemical degradation. The chains were purified individually and then recombined to form biologically active insulin. See Chance, R. E. et al, Diabetes Care 4:147-154 (1981).

Although the Goedell et al technique provides one method of producing human insulin, a more natual approach involves the biosynthesis of proinsulin, which is the immediate precursor of insulin. An advantage of producing insulin from proinsulin is the economy of fermenting and processing only one recombinant organism. The Goeddell et al technique requires the use of two recombinant organisms. Numerous researches have shown that proinsulin will oxidize spontaneously to form the correct disulfide bonds and can be quantitatively converted to insulin by controlled digestion with trypsin and carboxypeptidase B. See Steiner, D. and Clark, J. Proc. Nat. Acad. Sci., USA 60:622-629 (1968); and Kemmler, W. et al, J. Biol. Chem., 246:6786-6791 (1971). Gilbert and his co-workers demonstrated the feasibility of the proinsulin approach when they constructed plasmids in which most of the coding sequences of rat preproinsulin were fused to portions of the prepeptide region of penicillinase. When transformed into E.coli, some of these constructs produced fused preproteins that were correctly cleaved to rat proinsulin and segregated into the periplasmic space of the host organism. Analysis of the Gilbert plasmids shows that they contained fusions involving portions of both the penicillinase and the preproinsulin leader sequences. These were usually connected by short interposed sequences derived from the construction process and not related to either presequence. See Talmadge, K.et al, Proc.Nat.Acad. Sci., USA 77:3988-3992 (1980).

Although the Gilbert et al experiments demonstrate the feasibility of using the prepoinsulin approach to achieve expression of eukaryotic rat insulin, they do not teach how to create a recombinant plasmid carrying the human proinsulin gene in expressible form. Bell, G., et al, Nature 282:525-527 (1979) successfully cloned and sequenced the human preproinsulin gene. However, their work does not demonstrate successful expression of the human gene. European Patent Application 80303195.4, filed in the name of The Regents of the University of California, incorporates some of the work disclosed in the Bell et al publication, supra. However, the content of this application does not demonstrate actual expression of the human gene and does not indicate how to obtain an actual plasmid capable of such expression and samples thereof. Furthermore, it does not demonstrate that the human preproinsulin gene product may be properly processed by a suitably transformed bacterial host.

Therefore it is an object of the present invention to create a recombinant plasmid carrying the gene for human proinsulin in expressible form and to utilize such creation.

The invention provides a cloning vector for producing human preproinsulin through expression of DNA by a transformed host organism comprising a plasmid capable of transforming a host organism and having a preproinsulin coding DNA sequence including a functional transport coding DNA presequence fused to a structural gene coding for human proininsulin, said preproinsulin coding DNA sequence being under the control of an operator, promoter and ribosome binding site sequence and being positioned in the vector in correct reading frame with respect to the initiation codon for the functional transport coding DNA presequence so that a preproinsulin product is expressed by a host organism transformed by said cloning vector.

Novel Plasmid pJW2172 (ATCC 31891) and its progeny are included in the invention.

Preferably, the invention provides a host organism transformed by such a vector to express human proinsulin as a preproinsulin product that will be processed to human proinsulin by the host and then transported across the host's cell membrane.

Further provided by the invention is a process either (a) for preparing a cloning vector for producing human preproinsulin, or (b) for preparing an organism or culture capable of expressing a preproinsulin peptide product, or (c) for preparing preproinsulin or human proinsulin, or (d) for preparing human insulin; which process comprises: (a) providing a human preproinsulin cDNA sequence (including a functional transport presequence fused to a structural gene coding for human proinsulin) in a parent vector capable of becoming a functional part of a host organisms genetic material, said preproinsulin sequence being positioned to be under the control of an operator, promoter and ribosome binding site sequence and in correct reading frame with respect to the initiation codon for the functional transport presequence of said sequence; and, optionally, (b) transforming, with said cloning vector, the host organism and optionally cloning said organism to produce a transformant culture; and, optionally, (c) growing said transformed host organism or transformant culture under conditions suitable for expression of a DNA sequence coding for a preproinsulin peptide product and recovering the expressed preproinsulin peptide from the host cells and optionally converting said peptide to human proinsulin, or, if the organism is capable of correctly processing expressed preproinsulin to human proinsulin _in vivo_ and transporting the resulting human proinsulin across the host's cell membrane, allowing said transformed host organism or transformant culture to process the expressed preproinsulin peptide to human proinsulin _in vivo_, allowing human proinsulin to accumulate outside the host's cell membrane and recovering accumulated human proinsulin; and, optionally, (d)converting said human proinsulin to human insulin _in vitro_ and purifying said insulin.

The invention will now be further described and illustrated by way of the following description, taken in connection with the accompanying drawings wherein:

FIGURE 1 shows the distribution of immunoreactivity after gel filtration of osmotic shockates from bacterial cultures containing plasmid pJW2172. Elution positions of standard proteins are also indicated.

FIGURE 2 shows the distribution of radioactivity measured in slices obtained from tube gel SDS electrophoresis of an aliquot of $^{125}$I-labeled immunoprecipitated plasmid pJW2172 product. The vertical bar indicates the position of the stained band of bovine proinsulin.

FIGURE 3 shows the distribution of radioactivity obtained from automated sequential Edman degradation of $^{125}$I-labeled proinsulin product of plasmid pJW2172.

FIGURE 4 shows the distribution of radio-

- 7 -

activity obtained from automated sequential Edman degradation of $^{35}$S-labeled proinsulin product of plasmid pJW2172.

FIGURE 5 shows the binding of $^{125}$I-labeled plasmid pJW2172 immunoprecipitated proinsulin to a human C-peptide antiserum. Samples were tested with normal rabbit serum (N) and human C-peptide antiserum (C).

Very generally, the invention involves construction of novel recombinant plasmids carrying the human proinsulin structural gene in expressible form. Immediately preceeding the eukaryotic proinsulin structural gene on the plasmid is a functional transport presequence. The presequence can be comprised entirely of a prokaryotic bacterial transport presequence, entirely of a eukaryotic transport presequence, or of any functional combination of the two presequence types. The eukaryotic proinsulin structual gene sequence is oriented in the plasmid so that it is in correct translational reading frame with respect to the initiation codon of the functional transport presequence. Such an orientation allows a preproinsulin product to be synthesized by actively metabolizing transformed host organisms. The preproinsulin product is correctly processed to human proinsulin by the transformed host organisms, which then transport the human peptide across the cell membrane. In a transformed E. coli host, the human proinsulin accumulates in the bacterial periplasmic space. Following recovery, in vitro methods can be used to convert the human proinsulin to human insulin. See Steiner and Clark, supra, and Kemmler et al, supra. Alternatively, the recombinant plasmids could be used to transform host organisms that are not capable of correctly processing preproinsulin to human proinsulin in vivo. In that case the preproinsulin peptide would be recovered by disrupting the transformed hosts' cell walls. The transport sequence portion of the preproinsulin peptide could be removed enzymatically. See generally, Zwizinski, C. and Wickner, W., J. Biol. Chem., 255:7973 7977 (1980). In vitro methods could then be used to convert human proinsulin to human insulin. See Steiner and Clark, supra and Kemmler et al, supra.

More specifically, to illustrate the invention, construction of a representative recombinant plasmid is disclosed. This plasmid, designated as plasmid pJW2172, has been deposited with the American Type Culture Collection, Rockville, Maryland, 20852, USA. It has been awarded ATCC number 31891. Plasmid pJW2172 was constructed by modifying a parental plasmid, plasmid pHn677, which in turn was derived from the well-characterized plasmid pBR322 (ATCC 37017). Parental plasmid pHn677, which contains human preproinsulin cDNA cloned into the Pst I site of the ampicillinase gene of plasmid pBR322, was modified with a restriction endonuclease and a double stranded exonuclease. Large portions of the ampicillinase coding region were excised by these enzymes, resulting in the production of a variety of fused gene segments. Many of these generated proteins were detectable with insulin or C-peptide antisera. One such fused gene segment generating these detactable proteins is carried by plasmid pJW2172. Plasmid pJW2172 contains a perfect fused hybrid gene segment consisting of the aminoterminal coding half of the leader sequence of the prokaryotic ampicillinase (residues -23 to -12) fused to the eukaryotic human preproinsulin prepeptide beginning at residue -13. Expression of the fused segment results in the synthesis of a preproinsulin product, in vivo processing of the preproinsulin product and finally, and secretion of human proinsulin into the periplasmic space of E. coli host organisms transformed by plasmid pJW2172.  Characterization of the recovered human proinsulin product shows that it contains the A and B chain regions of insulin as well as specific C-peptide immunodeterminants.  Tryptic digestion can be used in vitro to convert proinsulin to insulin.  See Steiner and Clark, supra, and Kemmler et al, supra.

Parental plasmid, pHn677, was obtained originally by cDNA cloning of mRNA isolated from human insulinoma.  The cDNA was inserted into the Pst site of pBR322 by means of dC-dG tailing.  Plasmid pHn677 contains all of the proinsulin sequence plus the coding  sequence for 16 amino acids of the presequence. Although the DNA sequence in the parental plasmid was in the right orientation with respect to the beta-lactamase promoter, it was in the wrong reading frame with respect to the initiation codon of the beta-lactamase gene.  As a result there was no expression of the DNA sequence coding for the human proinsulin gene.

Therefore, in order to construct expression plasmids having the cDNA sequence in the correct reading frame, a restriction endonuclease and a double stranded exonuclease had to be used to remove most of the ampicillinase gene upstream from the preproinsulin cDNA sequence.  To accomplish this, both parental plasmid pHn677 and progenitor plasmid pBR322 had to be subjected to enzymatic digestion.

Plasmid pHn677 was digested with restriction endonuclease Pvu I.  The DNA was then treated with double stranded exonuclease Bal 31 to digest approximately 150 nucleotides from each end.  This DNA was further treated with restriction endonuclease Hind III. After subjecting the resulting DNA to gel electrophoresis on agarose gels, the larger set of DNA fragments was isolated from the gels by electroelution.

In a parallel procedure, Hinc II digested plasmid pBR322 DNA was subjected to Bal 31 to digest approximately 180 nucleotides from each end of the linearized plasmid. This DNA was then further digested with Hind III and Bam Hl. Finally the fragment containing the beta-lactamase promoter region was isolated from an acrylamide gel. This fragment was ligated to the Pvu l/Bal 31/Hind III treated large fragment. The ligation mixture was used to transform E. coli K12 strain CS412.

Transformants were first screened for tetracycline resistance. Some 350 clones exhibiting such resistance were further examined for insulin expression using the in situ radioimmunoassay procedure described by Broom, S. and Gilbert W., Proc. Nat. Acad. Sci., USA 75:2749 (1978). This procedure showed that 118 of the 350 clones might be expressing insulin. Therefore, these 118 clones were further assayed by means of a radioimmunoassay that utilized both the anti-insulin and the anti-C-peptide antibodies. The radioimmunoassays showed that at least 23 of these clones showed significant, i.e. greater than $\frac{1}{4}$ unit per mil of insulin activity.

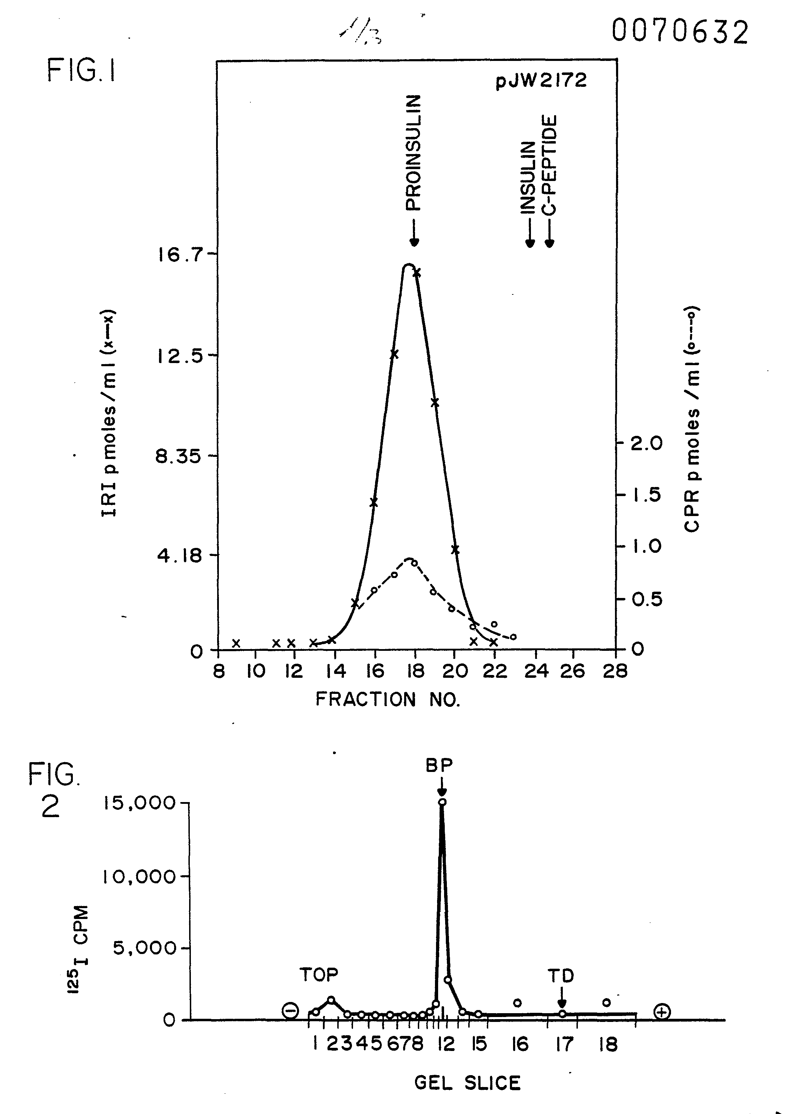

From the total series of plasmids containing a variety of hybrid bacterial-eukaryotic transport sequences attached to the human proinsulin sequence, one plasmid, designated as plasmid pJW2172, was selected for further study. As described in Chan, S. et al Proc. Nat. Acad. Sci., USA 78:5401-5405 (1981), and illustrated here in FIGURE 1, gel filtration of a concentrated osmotic shockate from cultures of transformed bacteria containing plasmid pJW2172 gave rise to a single homogeneous peak of C-peptide and insulin immunoreactivity which coeluted at the position of the proinsulin standard. Moreover, the ratio of C-peptide to insulin immunoreactivity of this component, i.e. approximately 1:15, corresponded well to the known cross-reactivity of human proinsulin in the highly specific human C-peptide immunoassay described by Faber, O.K., et al, _Diabetes_ 27:170-177 (1978).

These findings are readily explained by DNA sequence analysis of the fused region of plasmid pJW2172. As described more fully in Chan et al, _supra_, these studies revealed that fusion had occurred between residue -12 of the bacterial ampicillinase leader peptide sequence and residue -13 of the human presequence, thus creating a perfect hybrid leader sequence containing roughly half of each presequence without any substitution or modifications. This fused leader sequence preserves all of the structural features known to be required for export and cleavage. See Faber, et al, _supra_.

The experiments outlined in the Examples that follow demonstrate that plasmid pJW2172 generates a correctly cleaved secreted insulin product.

Example I - Characterization of the Protein
Product Secreted by Plasmid pJW2172

As described in Chan et al, _supra_, the proinsulin material generated by plasmid pJW2172 was characterized in the following matter. A sample of an osmotic shockate, from cultures of bacteria transformed by plasmid pJW2172, containing about 0.05 nM of the peptide was extracted with acid ethanol. The extract was then subjected to gel filtration on a column of Biogel P60 eluted with 2.5 M propionic acid. _See_ generally, Steiner, D., et al, in _Cell Biology: A Comprehensive Treatise_ 4:175-201 (1980). The tube containing the peak of insulin immunoreactivity, i.e., approximately 0.01 nM, was dried _in vacuo_ and divided into two equal aliquots.

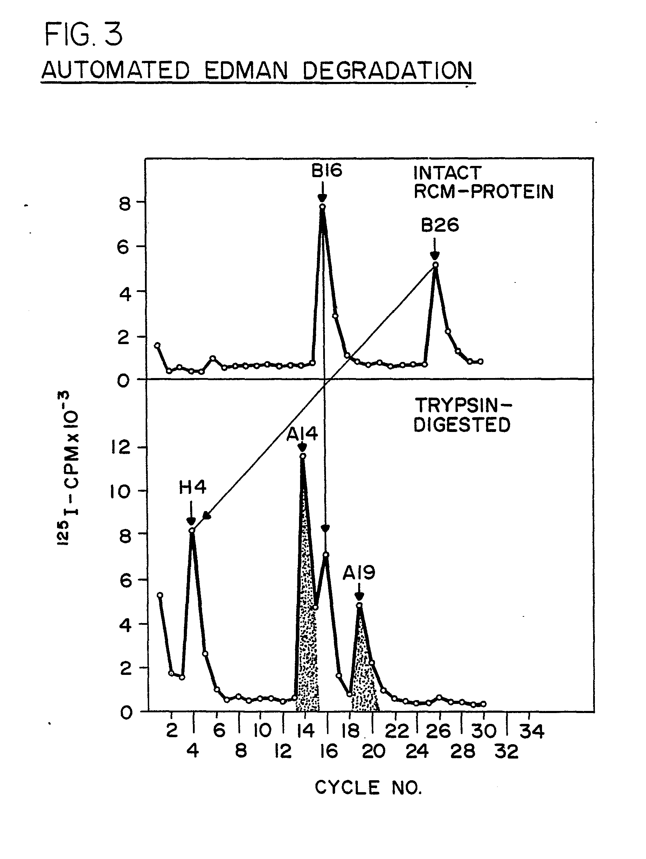

One aliquot was labeled by odination and then immunoprecipitated with insulin anti-serum. The resultant immunoprecipitate gave a single peak at the position of proinsulin when examined on a Biogel P30 column eluted with 3 M acetic acid. As shown in FIGURE 2, when run on tube gel SDS electrophoresis, the immunoprecipitate migrated as a single component having the same mobility as a bovine proinsulin standard.

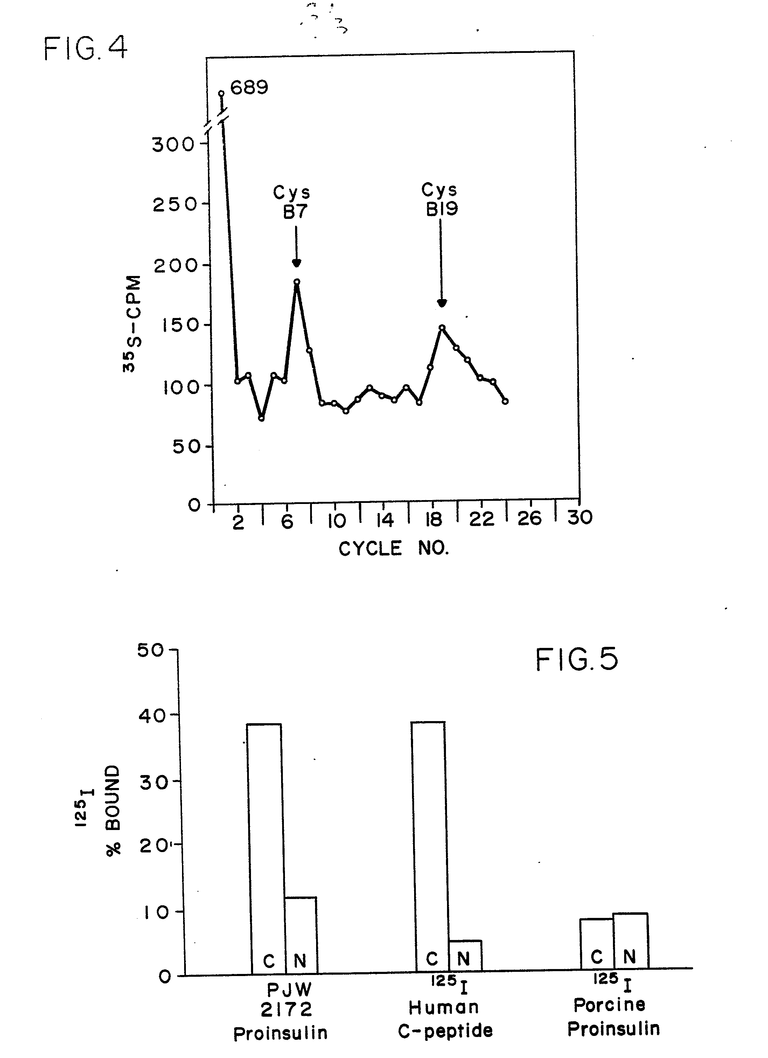

The second aliquot was used to determine whether cleavage of the presequence had occurred at the first residue of human proinsulin. The 0.5 pM aliquot of the immunoprecipitate was first reduced and carboxymethylated,, and then subjected to automated Edman degradation. See Chan et al, supra. The results are shown in FIGURE 3. They show that significant amounts of radioactively labeled tyrosine were found only at positions 16 and 26, as expected for human proinsulin. There was no indication of heterogeneity at the N-terminus. Further corroboration of this point was obtained when the osmotic shockate from a culture grown in the presence of $^{35}SO_4$ was similarly immuno-precipitated, gel filtered, reduced, carboxymethylated and then sequenced. These results are shown in FIGURE 4; they demonstrate the presence of the sulphur labeled B7 andB19 S-carboxymethyl-cysteine residues in correct register.

Example II - Characterization of the Proinsulin Like Peptide

Again, as described more fully in Chan et al, supra, to further characterize the proinsulin like peptide, three additional experiments were performed. In the first, an aliquot of the reduced and carboxymethylated iodinated immunoprecipitate was digested with trypsin and then submitted to automated Edman degradation. These results are shown in FIGURE 3. They demonstrate unequivocally that the protein contained the normal human insulin A chain, having tyrosines at position 14 and 19. In addition they show that trypsin had cleaved the B chain region at the arginine located at amino acid position 22, thus generating a heptapeptide that now contained the B26 tyrosine residue at position 4.

In the second experiment, the presence of C-peptide immunodeterminans in the aliquot material, which had originally been obtained by immunoprecipitation with an insulin antiserum, was assayed by binding to an antiserum against human C-peptide. These results are shown in FIGURE 5. They demonstrate that the carboxymethylated protein reacted as well as authentic iodinated human C-peptide. Porcine proinsulin did not bind significantly to this antiserum.

In a third experiment, the plasmid pJW2172 proinsulin product was treated with trypsin and carboxypepsidase B. This treatment converted the product to a component eluting at the position of insulin on gel filtration. There was no indication of the release of free A or B chain material.

On the basis of the evidence discussed in Examples I and II, it is clear that the final protein product generated by host bacteria transformed by plasmid pJW2172 is intact human proinsulin. Using techniques well known to those skilled in the art, it is possible to convert human proinsulin to human insulin _in_ _vitro_. _See_ Steiner and Clark, _supra_ and Kemmler et al, _supra_.

It may be seen therefore, that the invention involves construction of novel recombinant plasmids carrying the human proinsulin structural gene in expressible form. Immediately preceeding the eukaryotic structural gene on each plasmid is a functional transport presequence. The presequence can be comprised entirely of a prokaryotic bacterial transport presequence, entirely of a eukaryotic transport presequence, or of any functional combination of the two. Representative plasmid pJW2172 carries the human proinsulin structural gene attached to a functional fused hybrid transport presequence. The hybrid transport presequence is comprised of roughly half of the prokaryotic bacterial presequence fused to roughly half of the eukaryotic human presequence. The bacterial presequence codes for the amino-terminal end of the hybrid transport peptide; the human presequence codes for the carboxy-terminal end. E. coli host bacteria transformed by palsmid pJW2172 express a preproinsulin peptide product that is correctly processed to human proinsulin by the bacteria. This human proinsulin is transported by the bacteria across the cell membrane to the periplasmic space, where it accumulates. Following its recovery, in vitro methods such as tryptic digestion can be used to convert the human proinsulin to human insulin.

Various modifications in addition to those shown and described herein will become apparent to those skilled in the art from the foregoing description and accompanying drawings. Such modifications are intended to fall within the scope of the invention.

R E F E R E N C E S

Bell, G.I., Swain, W.F., Pictet, R.,Cordell, B., Goodman, H.M., and  Rutter, W.J., _Nature_ 282:525-527 (1979).

Broom, S., and Gilber, W., _Proc. Nat. Acad. Sci._, USA 75:2746-2749 (1978).

Chan, S.J., Weiss, J., Konrad, M., White, T., Bahl, C., Yu, S-D, Marks, D., and Steiner, D.F., _Proc. Nat. Acad. Sci._, USA 78:5401-5405 (1981).

Chance, R.E., Kroeff, E.P., Hoffmann, F.A., and Frank, B.H., _Diabetes Care_ 4:147-154 (1981)

Faber, O.K., Binder, C., Markussen, J., Heding, L.G., Naithani, V.K., Kuzuya, H., Blix, P., and Rubenstein, A.H., _Diabetes_ 27:170-177 (1978).

Goeddel, D.V., Kleid, G.G., Bolivar, F., Heyneker, H.L., Yansura, D.G., Crea, R., Hirose, T., Kraszewski, A., Itakura, K., and Riggs, A.D., _Proc. Nat. Acad. Sci._, USA 76:106-110 (1979)

Kemmler, W., Peterson, J.D., and Steiner, D.F., J. _Biol. Chem._, 246:6786-6791 (1971).

Permutt, M. and Kipnis, D.M., _Proc. Nat. Acad. Sci._, USA 69:506-509 (1972).

Steiner, D., and Clark, J., _Proc. Nat. Acad. Sci._, USA 60:622-629 (1968)

Steiner, D. F., Quinn, P.S., Patzelt, C., Chan, S.J., Marsh, J., and Tager, H.S., _Cell Biology: a Comprehensive Treatise_ 4:175-201 (1980).

Steiner, D., "Peptide Hormone Precursors", Peptide Hormones, Parsons, J.A., Editor, McMillan Press Limited, London, pp. 49-64 (1976).

Talmadge, K., Kaufman, J., and Gilbert, W., Proc. Nat. Acad. Sci., USA 77:3988-3993 (1980).

Zwizinski, C., and Wickner, W., J. Biol. Chem., 255: 7973-7977 (1980).

CLAIMS:

1. A cloning vector for producing human preproinsulin through expression of DNA by a transformed host organism comprising a plasmid capable of transforming a host organism and having a preproinsulin coding DNA sequence therein, said preproinsulin coding DNA sequence including a functional transport coding DNA presequence fused to a structural gene coding for human proinsulin, said preproinsulin coding DNA sequence being under the control of an operator, promoter and ribosome binding site sequence and being positioned in the vector reading frame with respect to the initiation codon for the functional transport coding DNA presequence so that a preproinsulin product is expressed by a host organism transformed by said cloning vector.

2. Plasmid pJW2172 (ATCC 31891) and its progeny.

3. A host organism transformed by the cloning vector of claim 1 or the plasmid of claim 2 or a transformant culture cloned from at least one said host organism.

4. A host organism or culture according to claim 3 which is Escherichia coli.

5. A host organism or culture according to claim 4 which is E. coli K12.

6. Preproinsulin which has been produced by a host organism or culture according to any one of claims 3 to 5.

7. A host organism or culture according to claim 3 which is capable of correctly processing expressed preproinsulin to human proinsulin in vivo and transporting the human proinsulin across the host's cell membrane.

8. Human proinsulin which has been produced by a host organism or culture according to claim 7 or which has been produced by in vitro conversion of preproinsulin according to claim 6.

9. Human insulin which has been produced by _in vitro_ conversion of human proinsulin according to claim 8.

10. A process either (a) for preparing a cloning vector for producing human preproinsulin, or (b) for preparing an organism or culture capable of expressing a preproinsulin peptide product, or (c) for preparing preproinsulin or human proinsulin, or (d) for preparing human insulin; which process comprises: (a) providing a human preproinsulin c DNA sequence (including a functional transport presequence fused to a structural gene coding for human proinsulin) in a parent vector capable of becoming a functional part of a host organisms genetic material, said preproinsulin sequence being positioned to be under the control of an operator, promoter and ribosome binding site sequence and in correct reading frame with respect to the initiation codon for the functional transport presequence of said sequence; and, optionally, (b) transforming, with said cloning vector, the host organism and optionally cloning said organism to produce a transformant culture; and, optionally, (c) growing said transformed host organism or transformant culture under conditions suitable for expression of a DNA sequence coding for a preproinsulin peptide product and recovering the expressed preproinsulin peptide from the host cells and optionally converting said peptide to human proinsulin, or, if the organism is capable of correctly processing expressed preproinsulin to human proinsulin _in vivo_ and transporting the resulting human proinsulin across the host's cell membrane, allowing said transformed host organism or transformant culture to process the expressed preproinsulin peptide to human proinsulin _in vivo_, allowing human proinsulin to accumulate outside the host's cell membrane and recovering accumulated human proinsulin; and, optionally, (d) converting said human proinsulin to human insulin _in vitro_

and purifying said insulin.

FIG.1

0070632

1/3

pJW2172

PROINSULIN

INSULIN

C-PEPTIDE

FIG. 2

BP

TOP

TD

FIG. 3

AUTOMATED EDMAN DEGRADATION

FIG.4

689

Cys B7

Cys BI9

$^{35}$S–CPM

300 250 200 150 100 50 0

2  6  10  14  18  22  26  30

CYCLE NO.

FIG.5

$^{125}$I % BOUND

50 40 30 20 10 0

C  N

C  N

C  N

PJW 2172 Proinsulin

$^{125}$I Human C-peptide

$^{125}$I Porcine Proinsulin